## Europäisches Patentamt

## European Patent Office

## Office européen des brevets

(19)

(11) Veröffentlichungsnummer: **0 181 414**
**A1**

(12) # EUROPÄISCHE PATENTANMELDUNG

(21) Anmeldenummer: **84113684.9**

(22) Anmeldetag: **13.11.84**

(51) Int. Cl.⁴: **C 07 C 23/02**
**C 07 C 17/02**

(43) Veröffentlichungstag der Anmeldung:
**21.05.86** Patentblatt **86/21**

(84) Benannte Vertragsstaaten:
**BE DE FR GB IT NL**

(71) Anmelder: **Chemische Fabrik Kalk GmbH**
**Kalker Hauptstrasse 22 Postfach 91 01 57**
**D-5000 Köln 91(DE)**

(72) Erfinder: **Eisenhauer, Gerhard, Dr.**
**Roonstrasse 81**
**D-4150 Krefeld(DE)**

(72) Erfinder: **Strang, Robert**
**Mattlener Weg 9**
**D-5000 Köln 71(DE)**

(54) Verfahren zur Herstellung von Hexabromcyclododecan hoher Reinheit.

(57) Die Erfindung betrifft ein Verfahren zur Herstellung von Hexabromcyclododecan hoher Reinheit. In einem Lösemittel-Gemisch aus Dioxan und einem gradkettigen oder verzweigten aliphatischen Alkohol mit 1 bis 6 C-Atomen wird 1,5,9-Cyclododecatrien mit Brom bei niedriger Temperatur umgesetzt. Nach Abtrennen des Hexabromcyclododecans kann ein Teil der Mutterlauge ausgeschleust, durch frisches Lösemittel-Gemisch ersetzt und als Reaktionsmedium in den Verfahrenskreislauf zurückgeführt werden.

Die Erfindung betrifft ein Verfahren zur Herstellung von Hexabromcyclododecan, das in hoher Reinheit und einer gleichbleibenden Qualität erhalten wird.

Hexabromcyclododecan hat sich als ein hervorragendes Brandschutzmittel für Kunststoffe, insbesondere für Polystyrolschäume und thermoplastische Kunststoffe sehr bewährt. Die Herstellung von Hexabromcyclododecan durch Bromierung von 1,5,9-Cyclododecatrien in geeigneten Lösemitteln ist bereits mehrfach beschrieben. So wird beispielsweise nach dem Verfahren der DE-PS 12 90 543 Cyclododecatrien-1,5,9 in äthanolischer Lösung mit Brom zu Hexabromcyclododecan umgesetzt und in guten Ausbeuten erhalten. Nach dem in der DE-PS 30 13 002 beschriebenen Verfahren wird ein Teil der bei der Hexabromcyclododecan-Produktion anfallenden äthanolischen Mutterlauge ausgeschleust und durch frisches Lösemittel ersetzt. In kontinuierlicher Verfahrensweise wird dabei ein Hexabromcyclododecan von gleichbleibender Qualität und hoher Reinheit erhalten. Dieses nahezu farblose Produkt entspricht voll den Anforderungen bei der Verwendung als Brandschutzmittel für Polystyrolschäume. Für die Verwendung als Brandschutzmittel für thermoplastische Kunststoffe ist dieses Hexabromcyclododecan jedoch ungeeignet, da es sich bei den Verarbeitungstemperaturen in einer Höhe von 190-200°C bereits teilweise thermisch zersetzt.

Ein Herstellungsverfahren zu finden, wobei ein Hexabromcyclododecan erhalten wird, das diesen hohen Verarbeitungstemperaturen ohne thermische Zersetzung standhält, ist Aufgabe der vorliegenden Erfindung.

Gegenstand der Erfindung ist ein Verfahren zur Herstellung von Hexabromcyclododecan von hoher Reinheit und gleichbleibender Qualität durch Umsetzung von 1,5,9-Cyclododecatrien mit Brom in polaren Lösemitteln bei niedriger Temperatur und Rückführung eines Teiles der Mutterlauge. Das Verfahren ist dadurch gekennzeichnet, daß als polares Lösemittel ein Gemisch von Alkohol und Dioxan verwendet wird. Von Vorteil haben sich aliphatische Alkohole mit 1 bis 6 C-Atomen gezeigt, die erfindungsgemäß in einem Mischungsverhältnis von Alkohol zu Dioxan von 60 : 40 bis 40 : 60, vorzugsweise 50 : 50, eingesetzt werden. Vorteilhaft hat sich in Bezug auf die Ausbeute erwiesen, nach dem Abtrennen des Hexabromcyclododecans einen Teil der Mutterlauge auszuschleusen und diesen ausgeschleusten Teil der Mutterlauge durch frisches Lösemittel-Gemisch zu ersetzen und als Reaktionsmedium wieder in den Verfahrenskreislauf zurückzuführen. Der ausgeschleuste Mutterlaugenanteil kann beispielsweise 20 bis 50 Vol-%, vorzugsweise 30 Vol-%, bezogen auf die Gesamtmenge des Lösemittelgemisches betragen.

Der erfindungsgemäßen Verwendung von Dioxan als Lösemittelbestandteil lag die Erkenntnis zugrunde, daß Dioxan mit Brom eine intermediäre Bindung eingeht unter Bildung von Dioxandibromid, das als Reagens für schonende und kontrollierte Bromierung bekannt ist. Einige Beispiele zur Bromierung aromatischer Amine sind in Amer.Soc. 75 (1953) S. 3596-97 beschrieben.

Zur Herstellung von Hexabromcyclododecan nach dem erfindungsgemäßen Verfahren wird in einem Reaktionsgefäß ein Gemisch von Alkohol, vorzugsweise Äthanol, und Dioxan in einem Vol.-Verhältnis von vorzugsweise gleichen Volumenteilen vorgelegt und unter Rühren durch zwei örtlich voneinander getrennten Dosiervorrichtungen gleichzeitig 1,5,9-Cyclododecatrien und Brom im Molverhältnis von mindestens 1 : 3 langsam zugefügt. Da von dem Dioxan nach beendeter Reaktion ein Teil des Broms intermediär gebunden bleibt, ist es erforderlich, einen Bromüberschuß von etwa 5 bis 10 Gew-%, bezogen auf die theoretische Menge, einzusetzen. Durch Kühlung wird die Reaktionstemperatur auf 15-25°C gehalten, und bei der anschließenden etwa 1-stündigen Nachreaktion eine Temperatur von 25°C aufrechterhalten.

Nach beendeter Reaktion und Nachreaktion wird das ausgefallene Hexabromcyclododecan durch Absaugen abgetrennt und mit frischem Lösemittel-Gemisch gut nachgewaschen. Das Produkt wird anschließend in Wasser aufgeschlämmt, mit verdünnter Alkalilösung leicht alkalisch eingestellt und bei Raumtemperatur 1 Stunde gerührt. Nach erneuter Abtrennung wird das Produkt mit Wasser neutral gewaschen und anschließend getrocknet. Die Ausbeute nach diesem ersten Ansatz beträgt etwa 70 % der Theorie.

Bei einem folgenden Ansatz, der auch in Serienfolge beliebig oft wiederholt werden kann, wird ein Teil der Mutterlauge des vorangegangenen Ansatzes, etwa ein Anteil von 20 bis 50 Vol-%, ausgeschleust, durch frisches Lösemittel-Gemisch der ursprünglichen Zusammensetzung ersetzt und in den Verfahrenskreislauf zurückgeführt. Die Produkte, die aus diesen Wiederholungs- und Folgeansätzen gewonnen werden, erbringen Ausbeuten von etwa 95 % der Theorie und keinerlei Qualitätsminderungen gegenüber dem Ansatz, der mit frischem Lösemittel-Gemisch hergestellt worden ist. Das auf diese Weise gewonnene Hexabromcyclododecan ist in geschmolzenem Zustand bei einer Temperatur von 190°C nahezu farblos und zeigt keine Zersetzung bei dieser Temperatur.

Das erfindungsgemäß hergestellte Hexabromcyclododecan zeigt in 30%iger Dimethylformamidlösung bei einer Schichtdicke von 2,001 cm und einem Filter S42E68 eine Lichtdurchlässigkeit von etwa 93 %. Dieser Wert von oberhalb 90 % Lichtdurchlässigkeit bleibt auch nach mehrfacher Wiederverwendung der Mutterlauge, von der jeweils ein Teil ausgeschleust und durch frisches Lösemittel-Gemisch ersetzt worden ist, konstant.

Mit den nachfolgenden Beispielen soll das erfindungsgemäße Verfahren näher erläutert werden.


Beispiel 1

In einem Reaktionsgefäß werden 500 Volumenteile (VT) Dioxan und 500 VT Äthanol vorgelegt und unter Rühren aus 2 örtlich voneinander getrennten Dosiervorrichtungen im Laufe von 4 Stunden gleichzeitig 216 Gewichtsteile (GT) 1,5,9-Cyclododecatrien und 672 GT Brom zugefügt. Die Reaktionstemperatur wird durch Außenkühlung auf 15-25°C gehalten. Bei der einstündigen Nachreaktion bleibt die Temperatur bei 25°C. Das entstandene Hexabromcyclododecan wird auf einer Nutsche abgesaugt und 3 mal mit jeweils 100 VT des ursprünglichen Lösemittel-Gemisches nachgewaschen. Der Filterkuchen wird in 1000 VT Wasser aufgeschlämmt, mit verdünnter NaOH-Lösung leicht alkalisch eingestellt und 1 Stunde bei Raumtemperatur gerührt. Nach erneutem Absaugen wird das Produkt mit Wasser neutral gewaschen und anschließend im Umlufttrockenschrank bei einer Temperatur von 55°C getrocknet.

Es werden 587 GT Hexabromcyclododecan mit einem Schmelzbereich von 171-185°C und einem Bromgehalt von 83,9 % erhalten.

Die Ausbeute beträgt 68,6 % der Theorie. Die Schmelze ist bei bei einer Temperatur von 190°C nahezu farblos und zeigt keine Zersetzung. Die Lichtdurchlässigkeit, gemessen in einer 30%igen Dimethylformamidlösung in einer Schichtdicke von 2,001 cm in einer Quarzküvette und einem Filter S42E68 ( = 42 nm ) beträgt 93 %.


Beispiel 2

Von der Mutterlauge aus der Hexabromcyclododecan-Herstellung entsprechend dem Beispiel 1 werden 700 VT zusammen mit 150 VT Dioxan und 150 VT Äthanol in einem Reaktionsgefäß vorgelegt und unter Rühren im Laufe von 4 Stunden bei einer Temperatur von 15-25°C 216 GT Cyclododecatrien und 672 GT Brom gleichzeitig aus 2 örtlich voneinander getrennten Dosiervorrichtungen zugefügt. Die Aufarbeitung erfolgt analog dem Beispiel 1.

Es werden 800 GT Hexabromcyclododecan mit einem Schmelzbereich von 173-186°C und einem Bromgehalt von 73,8 % erhalten. Die Ausbeute beträgt 93,5 % der Theorie. Die Schmelze ist bei einer Temperatur von 190°C hell und zeigt keine Zersetzung. Die Lichtdurchlässigkeit beträgt 92 %.


Beispiel 3

In einem Reaktionsgefäß werden 500 VT Dioxan und 500 VT Isobutanol vorgelegt und unter Rühren 216 GT Cyclododecatrien und 700 GT Brom gleichzeitig zudosiert. Die Reaktionsbedingungen und Aufarbeitung entsprechen dem Beispiel 1.

Es werden 646 GT Hexabromcyclododecan mit einem Schmelzbereich von 172-184 °C und einem Bromgehalt von 74,4 % erhalten. Die Ausbeute beträgt 75,5 % der Theorie. Die Schmelze ist bei einer Temperatur von 190 °C hell und zeigt keine
Zersetzung. Die Lichdurchlässigkeit beträgt 93 %.

## Beispiel 4

Von der Mutterlauge aus der Hexabromcyclododecan-Herstellung
entsprechend dem Beispiel 3 werden 700 VT zusammen mit 150
VT Dioxan und 150 VT Isobutanol in einem Reaktionsgefäß vorgelegt. 216 GT Cyclododecatrien und 700 GT Brom werden unter
den gleichen Bedingungen wie in den vorangegangenen Beispielen zur Reaktion gebracht und das Produkt in gleicher Weise
aufgearbeitet.

Es werden 775 GT Hexabromcyclododecan mit einem Schmelzbereich von 175-183 °C und einem Bromgehalt von 74,5 % erhalten. Die Ausbeute beträgt 90,5 % der Theorie. Die Schmelze
ist bei einer Temperatur von 190 °C hell und zeigt keine Zersetzung. Die Lichtdurchlässigkeit beträgt 91 %.

Patentansprüche

1. Verfahren zur Herstellung von Hexabromcyclododecan von hoher Reinheit und gleichbleibender Qualität durch Umsetzung von 1,5,9-Cyclododecatrien mit Brom in polaren Lösemitteln bei niedriger Temperatur und Rückführung eines Teiles der Mutterlauge, dadurch gekennzeichnet, daß als polares Lösemittel ein Gemisch von Alkohol und Dioxan verwendet wird.

2. Verfahren nach Anspruch 1, dadurch gekennzeichnet, daß als Alkohol gradkettige oder verzweigte aliphatische Alkohole mit 1 bis 6 C-Atomen verwendet werden.

3. Verfahren nach Ansprüchen 1 und 2, dadurch gekennzeichnet, daß das Mischungsverhältnis von Alkohol zu Dioxan 60 : 40 bis 40 : 60, vorzugsweise 50 : 50 beträgt.

4. Verfahren nach Ansprüchen 1 bis 3, dadurch gekennzeichnet, daß nach Abtrennen des Hexabromcyclododecans ein Teil der Mutterlauge ausgeschleust, durch frisches Lösemittel-Gemisch ersetzt und als Reaktionsmedium in den Verfahrenskreislauf zurückgeführt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A,D | DE-A-3 013 002 (CHEMISCHE FABRIK KALK)<br>--- | | C 07 C 23/02<br>C 07 C 17/02 |
| A | US-A-3 833 675 (J. NEWCOMBE et al.)<br>* Beispiel 10 *<br>--- | | |
| A | US-A-3 716 591 (D.G. ERADY)<br><br>----- | | |

| | | |
|---|---|---|
| | | **RECHERCHIERTE SACHGEBIETE** (Int. Cl 4)<br><br>C 07 C 23/00<br>C 07 C 17/00 |

Der vorliegende Recherchenbericht wurde für alle Patentansprüche erstellt.

| Recherchenort | Abschlußdatum der Recherche | Prüfer |
|---|---|---|
| DEN HAAG | 21-06-1985 | VAN GEYT J.J.A. |